# EUROPEAN PATENT APPLICATION

(11) **EP 4 585 604 A1**
(43) Date of publication of application: **16.07.2025**
(21) Application number: 23863116.2
(22) Date of filing: 01.09.2023
(51) Int. Cl.: C07H 21/04

(54) **NUCLEIC ACID PRODUCTION METHOD, METHOD FOR REMOVING IMPURITIES, AND NUCLEIC ACID HAVING LESS IMPURITIES**

(30) Priority: 07.09.2022 JP 2022142150
(71) Applicant: Nippon Shokubai Co., Ltd., Osaka-shi, Osaka 541-0043 (JP)
(72) Inventor: NOMURA Yumi, Suita-shi, Osaka 564-0034 (JP); YOTSUJI Keisuke, Suita-shi, Osaka 564-0034 (JP); TANAKA Yuki, Suita-shi, Osaka 564-0034 (JP)
(74) Representative: Hoffmann Eitle
(86) International application number: PCT/JP2023/032085
(87) International publication number: WO 2024/053578

(57) **Abstract**

The present invention is a nucleic acid production method and the like, the method including: (A) synthesizing a nucleic acid in which an amino group of a base moiety is protected with an acyl protecting group; (B) bringing the nucleic acid obtained in step (A) into contact with a nucleophile under a condition of a neutral or acidic pH; and (C) deprotecting the protecting group for the nucleic acid obtained in step (B). An embodiment of the present invention reduces the content of a by-product (B), which cannot be removed by a known technique, and can produce a nucleic acid with high purity.

## Description

### Technical Field

The present invention relates to a method for producing a nucleic acid, a method for removing an impurity, and a nucleic acid with a low impurity content.

### Background Art

Pharmacologically active ingredients of known marketed pharmaceutical products have been mostly small-molecule compounds. However, pharmaceutical products containing a medium-molecule pharmacologically active ingredient, such as a peptide or a nucleic acid, have also been increasingly marketed recently. In particular, a nucleic acid drug has a characteristic mechanism of action not found in other drug discovery seeds, such as an ability to act specifically by forming base pairs with a target mRNA, and thus are now highly promising drug discovery seeds for future development.

A nucleic acid used as a raw material for a nucleic acid drug is usually produced by chemical synthesis. Examples of literatures introducing the method for producing a nucleic acid include Patent Literature 1 below.

Patent Literature 1 relates to a deprotection method for an oligonucleotide. In the method, a solid phase-synthesized oligonucleotide is brought into contact with an organic amine or the like to cleave protecting groups without removing the oligonucleotide from the solid phase.

From the viewpoint of ensuring safety, pharmaceutical products are strictly quality-controlled for types and amounts of impurities under various regulations. Similarly, impurities are strictly controlled in production of a drug substance, and development of a technique for removing impurities generated in production of a nucleic acid as a drug substance is further desired.

Methods known for purifying a nucleic acid by removing an impurity include reverse-phase cartridge (RPC), reverse-phase high-performance liquid chromatography (RP HPLC), anion-exchange chromatography (AEX), and polyacrylamide gel electrophoresis (PAGE).

Patent Literature 2 describes purification of an oligonucleotide (claim 1, [0006], etc.) using a mixed-mode matrix containing a strong anion-exchange ligand, a strong cation-exchange ligand, and a hydrophobic ligand to remove an impurity including a non-complexed oligonucleotide and a failure sequence.

Patent Literature 3 describes purification of an oligonucleotide using hydrophobic interaction chromatography (HIC) to remove a process-related impurity, such as an n-1 impurity, a P=O impurity, an abasic impurity, a CNEt impurity, and an N+1 impurity (claim 1, [0006], etc.).

Patent Literature 4 describes purification of an oligonucleotide using two-phase mobile phase-stationary phase liquid-liquid chromatography to remove other oligonucleotides, such as shortmers and longmers (claim 1 and [0003]).

Patent Literature 5 describes purification of an oligonucleotide using a titratable anion-exchange composition to remove an impurity, such as a short oligonucleotide (claim 1, [0012], etc.).

Patent Literature 6 describes purification of an oligonucleotide using a porous carrier to remove an impurity (claim 1, etc.).

Non-Patent Literature 1 describes control and removal of a nonoligonucleotide process-related impurity (PRI) in the manufacturing of therapeutic oligonucleotides.

### Citation List

### Patent Literature

Patent Literature 1: JP 4705716 B
Patent Literature 2: JP 2022-539327 T
Patent Literature 3: JP 2019-518759 T
Patent Literature 4: JP 2014-525254 T
Patent Literature 5: JP 2005-520547 T
Patent Literature 6: JP H07-227284 A

### Non-Patent Literature

Non-Patent Literature 1: Org. Process Res. Dev., 2022, 26, 1130-1144

### Summary of Invention

### Technical Problem

The present inventors have found a problem in production of a nucleic acid: a nucleic acid (by-product (B)) with a mass 34 daltons greater than that of a target nucleic acid (A) is contained as an impurity in the nucleic acid (A). The by-product (B) has a molecular weight and physical properties very close to those of the nucleic acid (A) and thus cannot be removed using a known purification method, such as those in Patent Literatures 1 to 6 and Non-Patent Literature 1.

Thus, an object of the present invention is to provide a method for producing a nucleic acid, the method for removing the by-product (B) and reducing a content of an impurity. The present invention also provides a method for removing the by-product (B) and a nucleic acid having a low content of the by-product (B).

### Solution to Problem

As a result of intensive research, the present inventor has found that the content of the by-product (B) is predominantly reduced by synthesizing a nucleic acid, then treating the synthesized nucleic acid with a nucleophile when an amino group of a base moiety is protected with an acyl protecting group, and then deprotecting the protecting group. Based on the finding, the present inventor has completed the invention described below.
[1] A method for producing a nucleic acid, the method including:
   (A) synthesizing a nucleic acid in which an amino group of a base moiety is protected with an acyl protecting group;
   (B) bringing the nucleic acid obtained in step (A) into contact with a nucleophile under a condition of a neutral or acidic pH; and
   (C) deprotecting the protecting group of the nucleic acid obtained in step (B).
[2] The production method according to [1], in which the pH is from about 3 to about 8.
[3] The production method according to [1] or [2], in which the nucleophile is water and/or an alcohol.
[4] The production method according to any of [1] to [3], in which the contact is made with the nucleophile at from about 5 to about 80°C for about 0.25 hours or more.
[5] A method for removing a nucleic acid impurity from a target nucleic acid, the nucleic acid impurity being 34 daltons greater than the nucleic acid, the method including bringing a nucleic acid in which an amino group of a base moiety is protected with an acyl protecting group into contact with a nucleophile under a condition of a neutral or acidic pH, and then deprotecting the protecting group of the nucleic acid.
[6] The removal method according to [5], in which the pH is from about 3 to about 8.
[7] The removal method according to [5] or [6], in which the nucleophile is water and/or an alcohol.
[8] The removal method according to any of [5] to [7], in which the contact is made with the nucleophile at from about 5 to about 80°C for about 0.25 hours or more.
[9] A nucleic acid, in which, in mass spectroscopy, a detected ion intensity ratio of a nucleic acid impurity present in the nucleic acid and having a mass 34 daltons greater than that of the nucleic acid is 0.50 or less relative to the nucleic acid, when a detected ion intensity of the nucleic acid is taken as 100.

### Advantageous Effects of Invention

The method for producing a nucleic acid according to an embodiment of the present invention reduces the content of the by-product (B), which cannot be removed by a known technique, and can produce a nucleic acid with high purity.

### Description of Embodiments

Hereinafter, aspects related to a method for producing a nucleic acid according to an embodiment of the present invention will be described in detail. However, the following description is an example for explaining the present invention and is not intended to particularly limit the present invention to the scope of this description.

The term "about" used in the present description and claims means a numerical value within a range of ±10%, preferably a numerical value within a range of ±5%, more preferably a numerical value within a range of ±2%, and particularly preferably a numerical value within a range of ±1% of the numerical value indicated by "about".

### 1. Method for Producing Nucleic Acid

A method for producing a nucleic acid according to an embodiment of the present invention is a method for producing a nucleic acid, the method including:
(A) synthesizing a nucleic acid in which an amino group of a base moiety is protected with an acyl protecting group;
(B) bringing the nucleic acid obtained in step (A) into contact with a nucleophile under a condition of a neutral to acidic pH; and
(C) deprotecting the protecting group of the nucleic acid obtained in step (B).

### Nucleic Acid

A nucleic acid in an embodiment of the present invention has a structure in which nucleosides are linked by a phosphodiester bond, a thiophosphate ester bond, or an amide phosphate ester bond, and is preferably an oligonucleotide. The length of the oligonucleotide is not particularly limited but is, for example, from 10 to 100 bases and particularly preferably from 12 to 60 bases.

The nucleic acid in an embodiment of the present invention is a synthesized single-stranded nucleic acid in which a sugar is ribose or deoxyribose, and bases are selected from adenine (A), thymine (T), uracil (U), guanine (G), and cytosine (C). The nucleic acid in an embodiment of the present invention preferably contains a purine base, such as adenine. In the present description, the unit of "base length" may be replaced by the unit of "mer" or the unit of "nucleotide".

Examples of the nucleic acid in an embodiment of the present invention include DNA or RNA.

In the DNA, unless otherwise specified, the sugar moiety is a deoxyribose ring, and the base moiety is selected from adenine, thymine, guanine, and cytosine. In the RNA, unless otherwise specified, the sugar moiety is a ribose ring, and the base moiety is selected from adenine, uracil, guanine, and cytosine.

The single-stranded RNA or DNA synthesized according to an embodiment of the present invention can be used directly as an antisense, a CpG oligo, or an aptamer, and can also be used as an siRNA, an miRNA, a decoy, or a heteroduplex oligonucleotide (HDO) by forming base pairs (annealing) with another single-stranded RNA or DNA having a complementary base sequence to produce a doublestranded RNA or DNA.

### Modification of Nucleic Acid

In the nucleic acid in an embodiment of the present invention, for example, the base moiety may be modified with a substituent. Examples of the substituent include a halogen group, an acyl group, an alkyl group, an arylalkyl group, an alkoxy group, a hydroxy group, an amino group, a monoalkylamino group, a dialkylamino group, a carboxy group, a cyano group, and a nitro group. Examples of the modified base include an 8-bromoadenyl group, an 8-bromoguanyl group, a 5-bromocytosyl group, a 5-bromouracil group, a 5-iodouracil group, a 5-iodocytosyl group, a 5-fluorouracil group, a 5-methylcytosyl group (mC), an 8-oxoguanyl group, and a hypoxanthinyl group.

The nucleic acid in an embodiment of the present invention may be modified, for example, at the 2'-position or 5'-position of the sugar moiety or may have a cross-linking modification. Specific examples of the modification at the 2'-position include, for example, 2'-F, 2'-O-methyl (2'-OMe), and 2'-O-methoxyethyl (2'-MOE). Specific examples of the modification at the 5'-position include, for example, 5'-methyl (5'-Me) and 5'-cyclopropylene (5'-CP). Specific examples of the cross-linking modification include those in which a cross-linking structure is introduced between the 2'-position and the 4'-position, and examples include 2',4'-BNA/LNA, 2',4'-BNACOC, 2',4'-BNANC, ENA, AmNA, scpBNA, cEt, and GuNA.

The nucleic acid in an embodiment of the present invention can be modified in several nucleotide units (e.g., from 1 to 3 nucleotides) or in all nucleotide units.

### Method for Synthesizing Nucleic Acid

### (1) Step (A)

In step (A), a nucleic acid in which an amino group of a base moiety is protected with an acyl protecting group is synthesized.

Examples of the base having an amino group include adenine, guanine, cytosine, or bases obtained by modifying these bases with a substituent. Examples of the acyl protecting group include a benzoyl group, an isobutyryl group, an acetyl group, a phenoxyacetyl group, an isopropylphenoxyacetyl group, and a tert-butylphenoxyacetyl group, and preferably include a benzoyl group, an acetyl group, or an isobutyryl group.

Furthermore, in step (A), the phosphodiester bond, thiophosphate ester bond, or amide phosphate ester bond in the nucleic acid may be protected with a protecting group. Examples of the protecting group for the phosphodiester bond, thiophosphate ester bond, or amide phosphate ester bond include a protecting group used in a common procedure, for example, 2-cyanoethyl.

The method for synthesizing the nucleic acid in step (A) is not limited, and examples include a solid-phase synthesis method and a liquid-phase synthesis method. The method is preferably a solid-phase synthesis method or a liquid-phase synthesis method and particularly preferably a solid-phase synthesis method. Details of the solid-phase synthesis method will be described below as an example.

### Solid-Phase Synthesis Method

The solid-phase synthesis method in an embodiment of the present invention can be performed according to a common procedure unless otherwise specified in the present description. Examples of the solid-phase synthesis method include an H-phosphonate method, a phosphoester method, and a phosphoramidite method, and particularly preferably include a phosphoramidite method.

In step (A), the solid-phase synthesis method by a phosphoramidite method can be performed, for example, by sequentially performing steps (a) to (e) below.
(a) Deprotection step: removing a protecting group for a hydroxy group at the 5'-position of a nucleoside supported on solid phase via a linker.
(b) Coupling step: allowing condensation between a phosphorus atom attached to a hydroxy group at the 3'-position of the nucleoside phosphoramidite and a hydroxy group at the 5'-position of the nucleoside supported on the solid phase using an activator.
(c) Oxidation/sulfurization step: converting a phosphite ester bond (including a bond to which the protecting group is attached) between the nucleosides into a phosphodiester bond (including a bond to which the protecting group is attached) or a thiophosphate ester bond (including a bond to which the protecting group is attached) using an oxidizing agent or a sulfurizing agent.
(d) Capping step: attaching the protecting group to a hydroxy group at the 5'-position of the nucleoside supported on the solid phase, the hydroxy group not reacted in step (b), using a capping agent. The capping step is to prevent an unreacted group in step (b) from involving in reactions in subsequent steps (a) to (d).
(e) Cyanoethyl group removal step: repeating steps (a) to (d) a desired number of times, then removing the protecting group from the phosphodiester bond or thiophosphate ester bond between the nucleosides without detaching the produced nucleic acid with a desired chain length from the solid phase.

Steps (a) to (e) will be exemplified below using structural formulas.

The following structural formula is an example of a deoxyribose ring (a hydrogen atom attached to the 2'-position), but the same applies to the case of using a ribose ring (a hydroxy group at the 2'-position) or its modified product (e.g., a product in which the 2'-position is O-methoxyethylated (2'-MOE), O-methylated (2'-OMe), or fluorinated (2'-F)).

### Step (a):

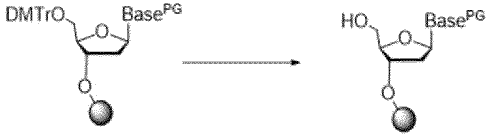

In the formula, DMTr represents a 4,4'-dimethoxytrityl group; Base^{PG} independently represents adenine, thymine, uracil, guanine, cytosine, or a modified form of these bases, and the amino group of the base moiety is protected with an acyl protecting group; and • part represents a solid-phase carrier attached to a linker.

Examples of the solid-phase carrier in the solid-phase synthesis include a glass bead, a resin bead, and silica gel, and the solid-phase carrier is preferably the glass bead or the resin bead.

Examples of the linker include 3-aminopropyl, succinyl, 2,2'-diethanolsulfonyl, and a long-chain alkylamino (LCAA).

For the protecting group for the hydroxy group at the 5'-position of a nucleoside, a nucleotide chain, a nucleoside phosphoramidite, or the like supported on solid phase, for example, a 4-methoxytrityl group or the like can be used in addition to a 4,4'-dimethoxytrityl group.

### Step (b):

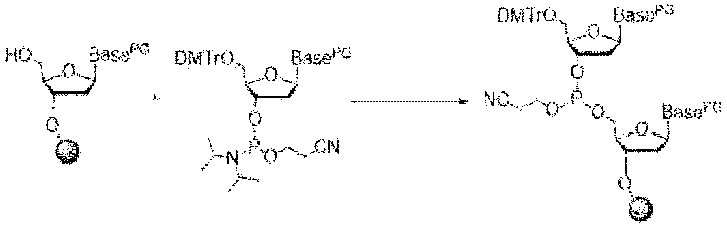

In the formula, DMTr, Base^{PG}, and • part are synonymous with those described above.

Examples of the activator used in step (b) include 1H-tetrazole, 5-ethylthiotetrazole, 4,5-dichloroimidazole, 4,5-dicyanoimidazole, benzotriazole triflate, imidazole triflate, pyridinium triflate, N,N-diisopropylethylamine, 2,4,6-collidine/N-methylimidazole, 5-(benzylthio)-1H-tetrazole, and 5-[3,5-bis(trifluoromethyl)phenyl]tetrazole.

### Step (c):

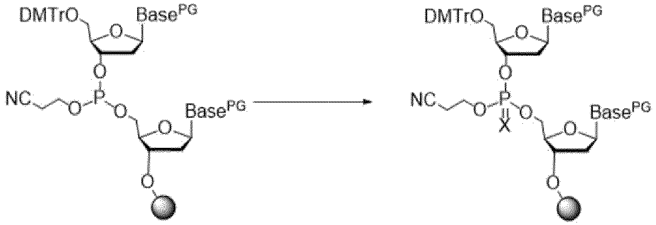

In the formula, DMTr, Base^{PG}, and • part are synonymous with those described above; and X represents an oxygen atom or a sulfur atom.

Examples of the oxidizing agent in step (c) include meta-chloroperbenzoic acid,
metaperiodate salt, hydrogen peroxide, iodide, and (1S)-(+)-(10-camphorsulfonyl)oxaziridine, and examples of the sulfurizing agent include 3-[(N,N-dimethylaminomethylidene)amino]-3H-1,2,4-dithiazole-5-thione (DDTT), 3H-1,2-benzodithiole-3-one-1,1-dioxide (Beaucage reagent), 3H-1,2-benzodithiole-3-one, bis(phenylacetyl) disulfide (PADS), tetraethylthiuram disulfide (TETD), and 3-amino-1,2,4-dithiazole-5-thione (ADTT). Examples of the reaction solvent in step (c) include dichloromethane, acetonitrile, pyridine, 3-picoline, water, tetrahydrofuran, or a solvent obtained by mixing these in any combination.

### Step (d):

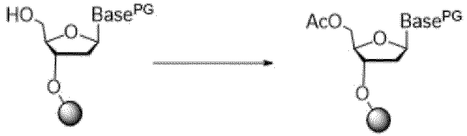

In the formula, DMTr, Base^{PG}, and • part are synonymous with those described above.

Examples of the capping agent in step (d) include acetic anhydride and phenoxyacetic anhydride. Examples of the reaction solvent in step (d) include acetonitrile, pyridine, 2,6-lutidine, tetrahydrofuran, or a solvent obtained by mixing these in any combination.

### Step (e):

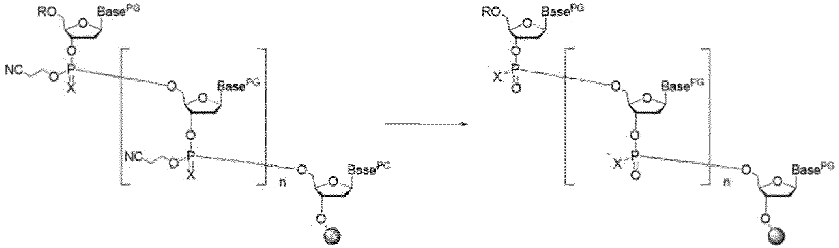

In the formula, Base^{PG}, **•,** and X are synonymous with those described above; R represents a 4,4'-dimethoxytrityl group or a hydrogen atom; and n represents an integer of 0 or more.

Examples of the cyanoethyl group-remover in step (e) include triethylamine, diethylamine, and 1,8-diazabicyclo[5.4.0]-7-undecene. The cyanoethyl group-remover can be diluted, for example, with acetonitrile, toluene, or the like and used.

### (2) Step (B)

In step (B), the nucleic acid obtained in step (A) is brought into contact with a nucleophile under a condition of a neutral or acidic pH. Step (B) reduces the content of the by-product (B), which cannot be removed by a known technique, and can produce a nucleic acid with high purity.

Examples of the nucleophile include, but are not particularly limited to, water, an alcohol, a thiol, an amine, a halide ion, and a cyanide ion. Specific examples include water, methanol, ethanol, n-propyl alcohol, isopropyl alcohol, butanol, pentanol, hexanol, heptanol, octanol, nonanol, decanol, undecanol, dodecanol, ethylene glycol, glycerol, phenol, methanethiol, ethanethiol, dodecanethiol, dithiothreitol, methylamine, dimethylamine, ethylamine, diethylamine, ethylenediamine, aniline, pyrrolidine, piperidine, piperazine, and morpholine. The nucleophile is preferably selected from water, alcohols, and thiols; examples more preferably include water and/or an alcohol and even more preferably include water, a primary alcohol, and a secondary alcohol. For example, the nucleophile is selected from water, methanol, ethanol, isopropyl alcohol, and n-butyl alcohol, and in terms of availability, ease of handling, and the like, the nucleophile is still more preferably selected from water, methanol, and ethanol, and is most preferably water. A liquid nucleophile may be used as a solvent.

Step (B) is performed under a condition of a neutral (e.g., pH from 6 to 8) or acidic (e.g., pH 6 or lower) pH, preferably under a condition of a pH of from about 3 to about 8, and more preferably under a condition of a pH of from about 4 to about 7.

The amount of contact between the nucleophile and the synthesized product in step (B) is not particularly limited, but from the viewpoint of production efficiency and the like, the amount is preferably from 1 mL to 1000 mL, more preferably from 5 mL to 100 mL, and particularly preferably from 10 mL to 50 mL per mmol of the nucleic acid. Longer contact time between the nucleophile and the synthesized product tends to enhance the effects of the present invention. The contact time is, for example, 0.25 hours or more, preferably 0.5 hours or more, more preferably 1 hour or more, even more preferably 2 hours or more, and from the viewpoint of production efficiency and the like, the contact time is desirably within 24 hours.

The temperature during the contact between the nucleophile and the synthesized product in step (B) is not particularly limited but is, for example, a temperature within the range of from 5°C to 80°C and more preferably of from 10°C to 50°C, and from the viewpoint of ease of operation and the like, the temperature is particularly preferably from 15°C to 30°C.

A material of a liquid-contacting surface of a vessel used during the contact with the nucleophile is not particularly limited, but examples include SUS316, SUS316L, SUS304, glass, an acrylic resin, polyethylene, polypropylene, polystyrene, PET, PTFE, PFA, ETFE, and FEP, and the material is preferably selected from glass, an acrylic resin, polyethylene, polypropylene, polystyrene, PET, PTFE, and PFA, and more preferably selected from glass, an acrylic resin, polyethylene, polypropylene, PTFE, and PFA. Even when the target oligonucleotide has a thiophosphate ester, performing the contact with the nucleophile in a non-metallic vessel can prevent cleavage due to the elution of a metal ion from the reaction vessel and the formation of a desulfurization product impurity (a product in which the thiophosphate ester bond of the target oligonucleotide is changed to a phosphodiester bond) during the deprotection step.

The contact with the nucleophile in step (B) can be made in a reaction column used for the synthesis or in another vessel to which the synthesized product is transferred after removal from the reaction column.

### (3) Step (C)

In step (C), the protecting group for the nucleic acid obtained in step (B) is deprotected.

When the method for synthesizing the nucleic acid in step (A) is a solid-phase synthesis method, step (C) (step of deprotecting the protecting group) can be simultaneously a step of cleaving the nucleic acid from the solid-phase carrier.

Following steps (a) to (e) described above of step (A) and step (B), step (f) below can be performed as step (C).

(f) Cleavage/deprotection step: cleaving the nucleic acid with a desired chain length produced up to steps (e) and (B) from the solid-phase carrier using a cleaving/deprotecting agent and removing the protecting group for the base moiety, a hydroxy group at the 2'-position, and the like.

### Step (f):

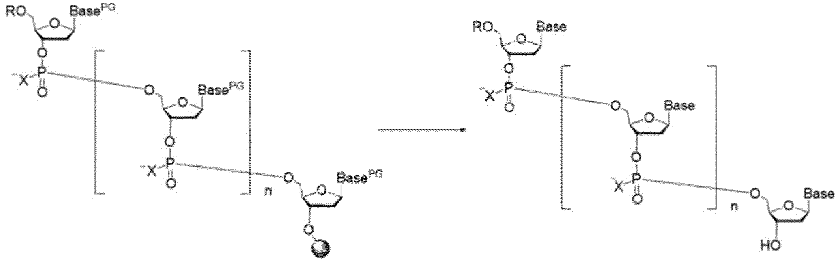

In the formula, Base^{PG}, **•,** and X are synonymous with those described above; Base independently represents adenine, thymine, uracil, guanine, and cytosine from which the protecting group is removed, or a modified form of these bases; R represents a 4,4'-dimethoxytrityl group or a hydrogen atom; and n represents an integer of 0 or more.

### Reaction Solution in Cleavage/Deprotection Step

Examples of the cleaving/deprotecting agent include basic substances, such as concentrated aqueous ammonia, methylamine, and sodium hydroxide. The ammonia concentration of the concentrated aqueous ammonia is preferably from 20 to 30 wt.% and more preferably from 25 to 30 wt.%. The amount of the cleaving/deprotecting agent to be used is preferably from 10 mL to 1000 mL per mmol of the nucleic acid supported on the solid-phase carrier and more preferably from 50 mL to 500 mL per mmol of the nucleic acid.

The cleaving/deprotecting agent can also be used by diluting, for example, with water, methanol, ethanol, isopropyl alcohol, 1-propyl alcohol, 1-butanol, 2-butanol, acetonitrile, tetrahydrofuran, 1,2-dimethoxyethane, dimethyl sulfoxide, or the like, and is preferably diluted with water, ethanol, or isopropyl alcohol. The reaction solution in the cleavage/deprotection step is preferably basic and more preferably has a pH of from 10 to 14.

The reaction temperature in step (f) is, for example, a temperature within the range of 5 to 75°C and preferably a temperature within the range of 15 to 60°C. The reaction time in step (f) is, for example, 48 hours or less, and the reaction is preferably performed in 24 hours or less.

The nucleic acid is cleaved from the solid-phase carrier by the cleaving/deprotecting agent, and furthermore, the protecting group attached to the nucleic acid is removed.

Steps (a) to (f) of the solid-phase synthesis method described above can be performed using a commercially available automated nucleic acid synthesizer or the like. In addition, according to the steps described above, a nucleic acid is produced so that the nucleotide chain extends from the carbon atom at the 3'-position toward the carbon atom at the 5'-position.

### (4) Separation and Purification Step

Nucleic acid purification can be performed on the produced nucleic acid using commonly performed separation and purification methods, including reverse-phase chromatography, reverse-phase ion-pair chromatography, ion-exchange chromatography, hydrophobic interaction chromatography, or gel filtration chromatography. Furthermore, a salt contained as an impurity in the solution can be removed by ultrafiltration or the like, and the oligonucleic acid solution can be powderized by freeze-drying.

### By-Product (B)

The method for producing a nucleic acid according to an embodiment of the present invention reduces the content of the by-product (B), which cannot be removed by a known technique, and can produce a nucleic acid with high purity. Specifically, in mass spectroscopy, a detected ion intensity ratio of a nucleic acid impurity (by-product (B)) with a mass 34 daltons greater than that of the target nucleic acid (A) is 0.50 or less and preferably 0.10 or less relative to the nucleic acid (A), when a detected ion intensity of the nucleic acid (A) is taken as 100.

The inventor of the present application presumes that during the synthesis procedure, mainly during step (a) described above, mutation causing high polarity occurs on the adenine base contained in the sequence, and this results in by-product (B) formation. In addition, the by-product (B) has physical properties, such as molecular weight and polarity, close to those of the target nucleic acid (A) and is difficult to remove by separation and purification techniques commonly used in nucleic acid production, such as anion-exchange chromatography. Thus, it is desirable to reduce the content of the by-product (B) in steps (B) and (C) before the separation and purification step.

Quality analysis of the produced nucleic acid can be performed by a commonly performed analysis of nucleic acid, for example, a combination of a separation method, such as reverse-phase chromatography, reverse-phase ion-pair chromatography, or ion-exchange chromatography, and a detector, such as a UV detector or a mass spectrometer. The amount of the by-product (impurity) in the nucleic acid can be calculated from a ratio of peak areas detected with a UV detector, an ion intensity ratio detected with a mass spectrometer, or the like. Several methods can be used to calculate the ion intensity ratio, including a method of calculating the ion intensity ratio after converting a detected multivalent ion group of the nucleic acid into a mass of a molecule (a neutral species) or m/z of a monovalent ion (a dimensionless quantity obtained by dividing a relative mass by a charge number of the ion, the relative mass obtained by dividing a mass of the ion by the unified atomic mass unit) by a deconvolution operation, or a method of calculating the ion intensity ratio by extracting the signal intensity of a specific multivalent ion.

### 2. Method for Removing Impurity

A method for removing an impurity according to an embodiment of the present invention is a method for removing a nucleic acid impurity (by-product (B)) from a target nucleic acid, the nucleic acid impurity with a mass 34 daltons greater than that of the nucleic acid, by bringing a nucleic acid in which an amino group of a base moiety is protected with an acyl protecting group into contact with a nucleophile under a condition of a neutral to acidic pH and then deprotecting the protecting group of the nucleic acid.

The method for removing an impurity according to an embodiment of the present invention can be performed by performing steps (B) and (C) described above.

### 3. Nucleic Acid with Low Impurity Content

A nucleic acid with low impurity content according to an embodiment of the present invention is a nucleic acid in which, in mass spectroscopy, a detected ion intensity ratio of a nucleic acid impurity present in the nucleic acid and having a mass 34 daltons greater than that of the nucleic acid is 0.50 or less relative to the nucleic acid, when a detected ion intensity of the nucleic acid is taken as 100.

The nucleic acid with low impurity content according to an embodiment of the present invention can be produced using the method for producing a nucleic acid according to an embodiment of the present invention described above.

### Examples

The present invention will be described in more detail with reference to examples and the like below, but the present invention is not limited to these examples and the like.

Unless otherwise specified, in DNA in examples and the like below, the sugar moiety is a deoxyribose ring, and the base moiety is selected from adenine, thymine, guanine, and cytosine. Unless otherwise specified, in LNA (2',4'-BNA) in the examples, the sugar moiety has a structure in which the oxygen atom at the 2'-position and the carbon atom at the 4'-position of the ribose ring are crosslinked (-CH₂-), and the base moiety is selected from adenine, thymine, guanine, and 5-methylcytosine. In production examples and comparative production examples, a benzoyl group was used as a protecting group for the amino group at the 6-position of adenine, an isobutyryl group was used as a protecting group for the amino group at the 2-position of guanine, and a benzoyl group was used as a protecting group for the amino group at the 4-position of cytosine and 5-methylcytosine.

### Production Example 1

### Study of Nucleophile Species

Using a SUS316L reaction column packed with a solid-phase carrier and a nucleic acid synthesizer OligoPilot (trade name) plus 100, phosphoramidites were sequentially condensed to a nucleoside attached to a solid-phase carrier via a linker, and finally a target oligonucleotide (FLP) was synthesized by passing a mixed solution of triethylamine/acetonitrile (1:1).

Then, the solid-phase carrier to which the target oligonucleotide was attached was immersed and shaken in 20 ml of any of the nucleophiles described in Table 1 per mmol of the target oligonucleotide supported on the solid-phase carrier in a glass test tube at 20°C for 3 hours. Thereafter, the solid-phase carrier was immersed in a mixed solution of a 28 wt.% aqueous ammonia solution/ethanol (4:1) at 60°C for 8 hours to cleave the target oligonucleotide from the solid-phase carrier and deprotect the protecting group.

After the cleavage/deprotection, the mixed solution was diluted with a mixed solution of water/ethanol (4: 1), and a crude solution of the target oligonucleotide was obtained (Examples 1 to 5).

The solid-phase carrier, the linker, and the target oligonucleotide are those shown below.
Solid-phase carrier, linker: Primer Support (trade name) 5G Unylinker 350 (Cytiva)
Target oligonucleotide (FLP): 5'-T(L)^G(L)^C^T^A^G^C^A^G^A^T^G^C^T^A(L)^mC(L)-3' (SEQ ID NO: 1: 16 mer, ^ represents a phosphothioate bond. In addition, each nucleoside unit with (L) described next to the base has the same structure as that of LNA (2',4'-BNA), and other nucleoside units represent the same structural unit as that of DNA. mC indicates that the base is methylcytosine.)

### Comparative Production Example 1

A crude solution of the target oligonucleotide was obtained by using the solid-phase carrier to which the target oligonucleotide synthesized in Production Example 1 was attached and performing the same operations under the same conditions as in Production Example 1 except for changing "Then, the solid-phase carrier to which the target oligonucleotide was attached was immersed and shaken in 20 ml of any of the nucleophiles described in Table 1 per mmol of the targeted oligonucleotide supported on the solid-phase carrier in a glass test tube at 20°C for 3 hours. Thereafter, the solid-phase carrier was immersed in a mixed solution of a 28 wt.% aqueous ammonia solution/ethanol (4: 1) at 60°C for 8 hours" to "Then, the solid-phase carrier to which the target oligonucleotide was attached was immersed in a mixed solution of a 28 wt.% aqueous ammonia solution/ethanol (4:1) at 60°C for 8 hours" (Comparative Example 1).

### Production Example 2

### Comparative Study of Contact Conditions

A crude solution of the target oligonucleotide was obtained by performing the same operations under the same conditions as in Production Example 1 except for changing "was immersed and shaken in 20 ml of any of the nucleophiles described in Table 1 per mmol of the targeted oligonucleotide supported on the solid-phase carrier in a glass test tube at 20°C for 3 hours" to "was immersed and shaken in water in a glass test tube under conditions of any of the contact temperature, water contact amount per amount of the targeted oligonucleotide supported on the solid-phase carrier, and contact time described in Table 2" (Examples 6 to 12).

### Comparative Production Example 2

A crude solution of the target oligonucleotide was obtained by using the solid-phase carrier to which the target oligonucleotide synthesized in Production Example 2 was attached and performing the same operations under the same conditions as in Comparative Production Example 1 (Comparative Example 2).

### Production Example 3

### Study of Contact Time

Using a SUS316L reaction column packed with a solid-phase carrier and a nucleic acid synthesizer OligoPilot (trade name) plus 100, phosphoramidites were sequentially condensed to a nucleoside attached to a solid-phase carrier via a linker, and finally a target oligonucleotide (FLP) was synthesized by passing a mixed solution of triethylamine/acetonitrile (1:1). Then, 960 mL of water per mmol of the target oligonucleotide supported on the solid-phase carrier was passed through the reaction column for 4 minutes to bring the solid-phase carrier into contact with water.

Then, the solid-phase carrier to which the target oligonucleotide was attached was immersed in a mixed solution of a 28 wt.% aqueous ammonia solution/ethanol (4:1) in a glass test tube at 60°C for 19 hours to cleave the target oligonucleotide from the solid-phase carrier and deprotect the protecting group.

After the cleavage/deprotection, the mixed solution was diluted with a mixed solution of water/ethanol (4:1), and a crude solution of the target oligonucleotide was obtained (Reference Example 1).

The solid-phase carrier, the linker, and the target oligonucleotide are those shown below.
Solid-phase carrier, linker: Primer Support (trade name) 5G Unylinker 350 (Cytiva)
Target oligonucleotide (FLP): 5'-C^T^A^G^C^A^G^A^T^G^C^T-3' (SEQ ID NO: 2: 12-mer DNA, ^ represents a phosphothioate bond)

### Comparative Production Example 3

A crude solution of the target oligonucleotide was obtained by performing the same operations under the same conditions as in Production Example 3 except for changing "a target oligonucleotide (FLP) was synthesized by passing a mixed solution of triethylamine/acetonitrile (1:1). Then, 960 mL of water per mmol of the target oligonucleotide supported on the solid-phase carrier was passed through the reaction column for 4 minutes to bring the solid-phase carrier into contact with water" to "a target oligonucleotide (FLP) was synthesized by passing a mixed solution of triethylamine/acetonitrile (1:1)." (Comparative Example 3).

### Production Example 4

### Purification of Target Oligonucleotide

A crude solution (using water as the nucleophile, Example 13) obtained by the same operations under the same conditions as in Production Example 1 and a crude solution (Comparative Example 4) obtained by the same operations under the same conditions as in Comparative Production Example 1 were prepared.

The crude solutions were each fractionated and purified using anion-exchange chromatography under the following conditions, and the purified oligonucleotide-containing solutions were obtained (Example 13 and Comparative Example 4).

Fractionation and purification conditions: ion-exchange resin: BioPro IEX SmartSep Q20 (YMC); column volume: 7 mL; column temperature: room temperature; mobile phase A: a mixed solution of 10 mM sodium hydroxide aqueous solution/methanol (85:15); mobile phase B: a mixed solution of 10 mM sodium hydroxide-2 M sodium chloride aqueous solution/methanol (85:15)

### Test Example 1: Measurement of By-Product (B) Amount

The crude solution of each oligonucleotide produced in Production Examples 1 to 4 and Comparative Production Examples 1 and 2 (and the purified oligonucleotide-containing solution in Production Example 4) was measured by ultra-high performance liquid chromatograph-mass spectroscopy (UHPLC-MS) under the conditions below to determine the amount of the target oligonucleotide (FLP) and the amount of the by-product (B) with a mass 34 daltons greater than that of the target oligonucleotide, and the content of the by-product (B) (ionic strength of by-product/ionic strength of target oligonucleotide (FLP) (%)) was calculated. The by-products (B) in the present description all have a mass 34 daltons greater than that of the target oligonucleotide, but the by-products (B) are different substances for each type of target oligonucleotide.

Measurement conditions: UHPLC instrument; Vanquish UHPLC System, mass spectrometer; Q Exactive, column; Waters ACQUITY UPLC Oligonucleotide BEH C18, 130 Å, 1.7 µm, 2.1 mm x 100 mm, UV detection; 260nm, Buffer A; 100 mM HFIP, 8 mM TEA in H₂O:MeCN = 98:2, Buffer B; H₂O:MeCN = 50:50, temperature; 60°C, ionisation method; ESI, detection mode; negative, spray voltage; 2.5 kV, capillary temperature; 250°C

The results measured above are as shown in Tables 1 to 4 below.

### Test Example 2: Measurement of Amount of Target Oligonucleotide (FLP)

Each sample solution of the crude solution of the oligonucleotide and the purified oligonucleotide-containing solution that are produced in Production Example 4 was measured by ultra-high performance liquid chromatography (UHPLC) under the following conditions, and the purity of the target oligonucleotide (FLP) (peak area of target oligonucleotide (FLP)/total area of all peaks (%)) was calculated.

Measurement conditions: UHPLC instrument; ACQUITY UPLC, column; Waters ACQUITY UPLC (trade name) Oligonucleotide BEH C18, 130 Å, 1.7 µm, 2.1 mm x 100 mm, UV detection; 260 nm, Buffer A; 100 mM HFIP, 8 mM TEA in H₂O:MeCN = 98:2, Buffer B; H₂O:MeCN = 50:50, temperature; 60°C

Furthermore, each sample solution of the crude solution of the oligonucleotide and the purified oligonucleotide-containing solution that are produced in Production Example 4 was diluted with water, then the absorbance was measured with a spectrophotometer, and the yield (a ratio of an OD value obtained by actual measurement to a theoretically obtainable maximum OD value) was calculated from the obtained OD values.

The results measured above are as shown in Table 4 below.

**[Table 1]**

| | Type of nucleophile | By-product (B) amount/FLP amount (%) |
|---|---|---|
| Example 1 | Water | 0.04 |
| Example 2 | Methanol | 0.02 |
| Example 3 | Ethanol | 0.09 |
| Example 4 | Isopropyl alcohol | 0.46 |
| Example 5 | N-butanol | 0.26 |
| Comparative Example 1 | None | 0.99 |

**[Table 2]**

| | Water contact temperature (°C) | Water contact time (hr) | Water contact amount (mL/mmol) | By-product (B) amount/FLP amount (%) |
|---|---|---|---|---|
| Example 6 | 15 | 2 | 10 | 0.2 |
| Example 7 | 15 | 8 | 7 | 0.08 |
| Example 8 | 15 | 8 | 31 | 0.08 |
| Example 9 | 20 | 0.5 | 23 | 0.44 |
| Example 10 | 20 | 2 | 26 | 0.16 |
| Example 11 | 30 | 0.5 | 5 | 0.09 |
| Example 12 | 30 | 3 | 18 | 0.01 |
| Comparative Example 2 | None | None | None | 0.69 |

**[Table 3]**

| | Whether water was passed | By-product (B) amount/FLP amount (%) |
|---|---|---|
| Comparative Example 3 | No | 1.11 |
| Reference Example 1 | Yes | 0.82 |

**[Table 4]**

| | Contact with water | Crude solution | | Purified solution | | Yield (%) |
|---|---|---|---|---|---|---|
| | | FLP purity (%) | By-product (B) amount/FLP amount (%) | FLP purity (%) | By-product (B) amount/FLP amount (%) | |
| Comparative Example 4 | No | 71.27 | 0.69 | 97.12 | 0.50 | 51 |
| Example 13 | Yes | 71.17 | 0.04 | 96.61 | 0.05 | 52 |

The by-product (B) amount/FLP amount (%) shown in Tables 1 to 4 is a ratio (%) of the by-product (B) when the full length product (FLP: target oligonucleotide) is taken to be 100 (%).

Table 1 shows that those (Examples 1 to 5) brought into contact with the specific nucleophile (water, methanol, ethanol, isopropyl alcohol, or n-butanol) according to an embodiment of the present invention had a significantly lower content of the by-product (B) than the one (Comparative Example 1) not brought into contact with the nucleophile.

Meanwhile, in the case of Example 2, in which methanol was used as the nucleophile, it was confirmed that an alkoxy by-product in which the adenine base of the target oligonucleotide was replaced by a methoxy group was formed in an amount of 3.20% (the ratio when the target oligonucleotide was taken to be 100%). The alkoxy by-product is presumed to be separable from the target oligonucleotide by reverse-phase chromatography.

Table 2 shows, for the contact with water according to an embodiment of the present invention, that the amount of contact water does not affect the content of the by-product (B) (Examples 7 and 8).

In addition, it was shown that the content of the by-product (B) was significantly reduced by contact with water for 0.5 hours (Example 9) compared with the case without contact with water (Comparative Example 2). Furthermore, it was shown that the decreasing rate of the by-product (B) was greater when the target oligonucleotide was brought into contact with water for 0.5 hours (Example 9 in Table 2) than when the target oligonucleotide was brought into contact with water for only 4 minutes (Reference Example 1 in Table 3), and the decreasing rate was even greater when the target oligonucleotide was brought into contact with water for 2 hours (Example 10 in Table 2). The above results showed that the effect of reducing the content of the by-product (B) is enhanced with the extension of the contact time.

In addition, it was shown that when the contact temperature with water was 30°C (Example 11 in Table 2), the by-product (B) decreased faster than when the contact temperature was 15°C (Example 6 in Table 2) or 20°C (Example 9 in Table 2). The above results showed that the effect of reducing the content of the impurity is enhanced with an increase in the contact temperature.

As shown in Table 4, it was shown that the contact with water according to an embodiment of the present invention is more effective in reducing the content of the by-product (B) than common fractionation and purification operations. In addition, it was confirmed that whether the contact with water is made does not affect the FLP purity or yield.

### Industrial Applicability

An embodiment of the present invention contributes to the production of a high-quality nucleic acid drug with low impurity content.

## Claims

1. A method for producing a nucleic acid, the method comprising:
(A) synthesizing a nucleic acid in which an amino group of a base moiety is protected with an acyl protecting group;
(B) bringing the nucleic acid obtained in step (A) into contact with a nucleophile under a condition of a neutral or acidic pH; and
(C) deprotecting the protecting group of the nucleic acid obtained in step (B).

2. The production method according to claim 1, wherein the pH is from about 3 to about 8.

3. The production method according to claim 1 or 2, wherein the nucleophile is water and/or an alcohol.

4. The production method according to any of claims 1 to 3, wherein the contact is made with the nucleophile at from about 5 to about 80°C for about 0.25 hours or more.

5. A method for removing a nucleic acid impurity from a target nucleic acid, the nucleic acid impurity having a mass 34 daltons greater than that of the nucleic acid, the method comprising bringing a nucleic acid in which an amino group of a base moiety is protected with an acyl protecting group into contact with a nucleophile under a condition of a neutral or acidic pH, and then deprotecting the protecting group of the nucleic acid.

6. The removal method according to claim 5, wherein the pH is from about 3 to about 8.

7. The removal method according to claim 5 or 6, wherein the nucleophile is water and/or an alcohol.

8. The removal method according to any of claims 5 to 7, wherein the contact is made with the nucleophile at from about 5 to about 80°C for about 0.25 hours or more.

9. A nucleic acid, wherein, in mass spectroscopy, a detected ion intensity ratio of a nucleic acid impurity present in the nucleic acid and having a mass 34 daltons greater than that of the nucleic acid is 0.50 or less relative to the nucleic acid, when a detected ion intensity of the nucleic acid is taken as 100.
